(19) Europäisches Patentamt
European Patent Office
Office européen des brevets

(11) EP 2 963 048 B1

(12) **EUROPEAN PATENT SPECIFICATION**

(45) Date of publication and mention of the grant of the patent:
**18.11.2020 Bulletin 2020/47**

(21) Application number: **14756953.7**

(22) Date of filing: **25.02.2014**

(51) Int Cl.:
*C07H 1/00* (2006.01) *C07H 5/02* (2006.01)

(86) International application number:
**PCT/JP2014/054416**

(87) International publication number:
**WO 2014/132940 (04.09.2014 Gazette 2014/36)**

# (54) METHOD FOR PRODUCING ALPHA-HALO-TETRAACYL GLUCOSE

VERFAHREN ZUR HERSTELLUNG VON ALPHA-HALO-TETRAACYL-GLUCOSE

PROCÉDÉ DE PRODUCTION D’UN ALPHA-HALO-TÉTRAACYL-GLUCOSE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.02.2013 JP 2013036333**
**26.12.2013 JP 2013268649**

(43) Date of publication of application:
**06.01.2016 Bulletin 2016/01**

(73) Proprietor: **Mitsubishi Tanabe Pharma Corporation**
**Osaka 541-8505 (JP)**

(72) Inventors:
- **UEDA, Keita**
**Osaka-shi**
**Osaka 541-8505 (JP)**
- **HATSUDA, Masanori**
**Osaka-shi**
**Osaka 541-8505 (JP)**
- **HYODO, Isao**
**Osaka-shi**
**Osaka 541-8505 (JP)**
- **TANIMOTO, Kouichi**
**Osaka-shi**
**Osaka 541-8505 (JP)**

(74) Representative: **Vossius & Partner Patentanwälte Rechtsanwälte mbB**
**Siebertstrasse 3**
**81675 München (DE)**

(56) References cited:
**WO-A1-2011/047113 WO-A1-2012/140120**
**JP-A- 2007 518 683**

- **POLAT T ET AL: "Zinc triflate-benzoyl bromide: a versatile reagent for the conversion of ether into benzoate protecting groups and ether glycosides into glycosyl bromides", CARBOHYDRATE RESEARCH, PERGAMON, GB, vol. 338, no. 5, 14 February 2003 (2003-02-14), pages 447-449, XP004404426, ISSN: 0008-6215, DOI: 10.1016/S0008-6215(02)00481-0**
- **GIRI SANTOSH KUMAR ET AL.: ’Indium(III) Triflate -Mediated One-Step Preparation of Glycosyl Halides from Free Sugars’ SYNTHETIC COMMUNICATIONS vol. 40, no. 22, 2010, pages 3378 - 3383, XP055285548 DOI: 10.1080/00397910903419886**
- **LEMAIRE SEBASTIEN ET AL.: ’Stereoselective C-Glycosylation Reactions with Arylzinc Reagents’ ORGANIC LETTERS vol. 14, no. 6, 01 January 2012, pages 1480 - 1483, XP055069093 DOI: 10.1021/OL300220P**
- **PALLAVI TIWARI ET AL.: ’ACYLATION OF CARBOHYDRATES OVER A12O3: PREPARATION OF PARTIALLY AND FULLY ACYLATED CARBOHYDRATE DERIVATIVES AND ACETYLATED GLYCOSYL CHLORIDES’ CARBOHYDRATE RESEARCH vol. 341, no. 3, 27 February 2006, pages 339 - 350, XP025010362 DOI: 10.1016/J.CARRES.2005.11.035**
- **MO HUNSEN ET AL.: ’Mild one-pot preparation of glycosyl bromides’ CARBOHYDRATE RESEARCH vol. 340, no. 17, 12 December 2005, pages 2670 - 2674, XP055285561 DOI: 10.1016/J.CARRES.2005.09.016**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **WENG SHIUE-SHIEN ET AL.: 'Facile preparation of a-glycosyl iodides by in situ generated aluminum iodide: straightforward synthesis of thio-, seleno-, and O-glycosides from unprotected reducing sugars' JOURNAL OF CARBOHYDRATE CHEMISTRY vol. 29, no. 8-9, 01 January 2011, pages 429 - 440, XP055285562 DOI: 10.1080/07328303.2011.565894**
- **HIGASHI KUNIO ET AL.: 'Direct transformation of O-glycoside into glycosyl bromide with the combination of trimethylsilyl bromide and zinc bromide' CHEMICAL & PHARMACEUTICAL BULLETIN vol. 39, no. 10, 01 January 1991, pages 2502 - 2504, XP055285566 DOI: 10.1248/CPB.39.2502**
- **MURAKAMI TEIICHI ET AL.: 'Efficient synthesis of omega-mercaptoalkyl 1,2-trans-glycosides from sugar peracetates' CARBOHYDRATE RESEARCH vol. 342, no. 8, 11 June 2007, pages 1009 - 1020, XP022026765 DOI: 10.1016/J.CARRES.2007.02.024**
- **MURAKAMI TEIICHI ET AL.: 'Stereoselective glycosylations using benzoylated glucosyl halides with inexpensive promoters' CARBOHYDRATE RESEARCH vol. 343, no. 8, 09 June 2008, pages 1297 - 1308, XP022648101 DOI: 10.1016/J.CARRES.2008.03.019**
- **WANG QINGBING ET AL.: 'A facile preparation of peracylated alpha-aldopyranosyl chlorides with thionyl chloride and tin tetrachloride' CARBOHYDRATE RESEARCH vol. 343, no. 17, 24 November 2008, pages 2989 - 2991, XP025561370 DOI: 10.1016/J.CARRES.2008.08.037**

## Description

TECHNICAL FIELD

The present invention relates to a novel method for producing an α-halo-tetraacyl-glucose useful as a synthetic intermediate of a medicine.

BACKGROUND ART

An α-halo-tetraacyl-glucose which is a compound in which an acyl group is introduced into hydroxy groups of glucose, and a halogen atom is introduced on an anomeric carbon of the same, is a compound useful as a synthetic intermediate of a medicine. For example, it can be used for synthesis of 1-(β-D-glucopyranosyl)-4-methyl-3-[5-(4-fluorophenyl)-2-thienylmethyl]benzene (i.e., canagliflozin) which is used for treatment or prophylaxis of diabetes, etc. (see Patent Literatures 1, 2, and 3, and Non-Patent Literature 1).

In the above-mentioned Patent Literature 2, there is disclosed a preparation method of an α-halo-tetraacyl-glucose which is a key intermediate, which is constituted by the two step reactions that D-glucose and an acylating agent are reacted in the presence of a base to obtain a derivative in which whole hydroxy groups of the D-glucose are protected by acyl groups, and the hydroxy group on an anomeric carbon are substituted by a halogen atom to obtain an objective product.

As another method for producing an α-halo-tetraacyl-glucose, for example, a preparation method in which sugar and acetyl chloride or acetyl bromide are reacted in the presence of a base to prepare an α-halo-tetraacetyl sugar has been reported (see Non-Patent Literature 2). However, the acid halide and the base are used excessively (10 equivalents or more), so that this is not a sufficiently effective method as an industrial preparation method. Moreover, in the reaction with benzoyl chloride, an α-chloro-tetrabenzoyl glucose cannot be obtained. It has also been described that a secondary hydroxy group cannot be pivaloylated in this method.

Also, as another method for producing an α-halo-tetraacyl-glucose, a method for producing an α-halo-tetraacyl-glucose by treating glucose, wherein a hydroxy group at the anomer position is protected by methyl group and the other hydroxy groups are protected by acetyl group, with zinc halide and acetyl halide has been reported (see Non-Patent Literature 3). In this method, even though the glucose wherein the hydroxy groups are all protected is used as the starting material, excess amount of acetyl halide is necessary and zinc halide is used in amount of 0.25 molar equivalent. This is not a sufficiently effective method as an industrial preparation method.

Also, as a different method, a method has been reported in which benzoyl bromide is reacted with sugar in which the hydroxy group at an anomeric position is protected by methyl or para-methoxyphenyl, and all the other hydroxy groups are protected by benzyl to prepare an α-bromo-tetrabenzoyl sugar (see Non-Patent Literature 4). However, the starting material in this preparation method is ether protected sugar. Thus, when unprotected sugar is used as a starting material, the process takes plural steps.

As a still further different method, a method has been reported in which sugar and acid halide are reacted in the presence of indium triflate to obtain an α-halo-tetraacyl sutar (see Non-Patent Literature 5). However, indium triflate is not an inexpensive nor easily available Lewis acid, and there is disclosed when a metal halide which acts as a general Lewis acid such as zinc chloride, etc., is employed in this method, the Lewis acid is required to be used in a stoichiometric amount.

PRIOR ART LITERATURES

PATENT LITERATURES

[Patent Literature 1] WO 2005/012326A pamphlet
[Patent Literature 2] WO 2011/047113A pamphlet
[Patent Literature 3] WO 2012/140120A pamphlet

NON-PATENT LITERATURES

[Non-Patent Literature 1] Knochel, P., et al., "Stereoselective C-Glycosylation Reactions with Arylzinc Reagents", Organic Letters, 2012, vol. 14, No. 6, pp.1480-1483 [Non-Patent Literature 2] Tiwari, P., et al., "Acylation of carbohydrates over Al2O3: preparation of partially and fully acylated carbohydrate derivatives and acetylated glycosyl chlorides", Carbohydrate Research, 2006, vol. 341, pp. 339-350
[Non-Patent Literature 3] Grynkiewicz, G., et al., "Direct transformation of methyl glycopyranosides into corresponding Glycosyl Halides", Polish J. Chem., 1987, vol. 61, pp. 149-153
[Non-Patent Literature 4] Polat, T., et al., "Zinc triflate-benzoyl bromide: a versatile reagent for the conversion of ether into benzoate protecting groups and ether glycosides into glycosyl bromides", Carbohydrate Research, 2003, vol. 338, pp. 447-449 [Non-Patent Literature 5] Santosh, K. G., etc., "Indium(III) triflate-mediated one-step preparation of Glycosyl halides from free sugars", Synthetic Communications, 2010, vol. 40, pp. 3378-3383

SUMMARY OF THE INVENTION

PROBLEMS TO BE SOLVED BY THE INVENTION

According to the conventional method, an α-halo-tetraacyl-glucose which is a useful intermediate of a medicine, etc., cannot be prepared with good efficiency by introducing various kinds of acyl groups and halogen atoms depending on the purposes in one step into glucose which is inexpensive, so that there is a problem in the point of the production costs. An object of the present invention is to provide an efficient and excellent preparation method of an α-halo-tetraacyl-glucose suitable for industrial preparation.

MEANS FOR SOLVING THE PROBLEMS

The present inventors have intensively studied, and as a result, they have found a method for producing an α-halo-tetraacyl-glucose represented by the formula (III):

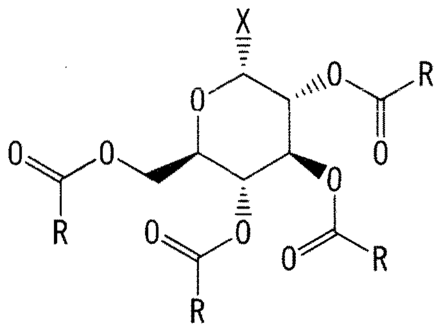

(III)

wherein R represents optionally substituted $C_{1-6}$ alkyl or optionally substituted aryl, and X represents a halogen atom, in one step with high yield by using unprotected D-glucose or unprotected $C_{1-6}$ alkyl D-glucoside as a starting material, and reacting it with an
acid halide in the presence of a catalytic amount of a Lewis acidic metal halide selected from the group consisting of zinc bromide, cobalt bromide and bismuth bromide,
whereby the present invention as defined in the claims has been accomplished.

EFFECTS OF THE INVENTION

According to the present invention, desired acyl groups and a desired halogen atom can be each introduced in one step into hydroxy groups and an anomeric carbon of unprotected D-glucose or unprotected $C_{1-6}$ alkyl D-glucoside which are inexpensive, and an α-halo-tetraacyl-glucose which is useful as an intermediate of a medicine, etc. can be prepared industrially advantageously. A reactive derivative of a carboxylic acid and a metal halide to be used in a disclosed aspect are inexpensive and easily available, and their amounts to be used are also a stoichiometric amount or a catalytic amount, so that the method is industrially advantageous.

BEST MODE FOR CARRYING OUT THE INVENTION

In the present invention, the lower alkyl is a linear and branched alkyl having 1 to 6 ($C_{1-6}$) carbon atoms. It is understood that this definition applies throughout to all instances where "lower alkyl" is referred to. More specifically, there may be mentioned methyl, ethyl, n-propyl, i-propyl, n-butyl, t-butyl, etc. These groups may be each substituted by an optional substituent(s).

The lower alkoxy may be mentioned linear and branched lower alkyl-O-having 1 to 6 ($C_{1-6}$) carbon atoms. More specifically, there may be mentioned methoxy, ethoxy, etc., and these groups may be each substituted by an optional substituent(s).

The halogen atom may be mentioned a fluorine atom, a chlorine atom, a bromine atom and an iodine atom.

The aryl may be mentioned a 6-10 membered aromatic carbocyclic group. More specifically, there may be mentioned phenyl and naphthyl, and phenyl is preferred. These groups may be each substituted by an optional substituent(s).

The aryloxy may be mentioned a 6-10 membered aromatic carbocyclic group-O-. More specifically, there may be mentioned phenoxy and naphthoxy, and phenoxy is preferred. These groups may be each substituted by an optional substituent(s).

The reactive derivative of a carboxylic acid may be mentioned, for example, an acid anhydride, an active ester and an acid halide, etc.

As the present invention, there is provided a method for producing an α-halo-tetraacyl-glucose represented by the formula (III):

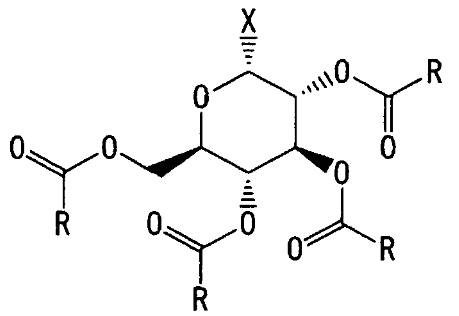

(III)

wherein R represents an optionally substituted $C_{1-6}$ alkyl or optionally substituted aryl, and X represents a halogen atom, which comprises reacting D-glucose or $C_{1-6}$ D-glucoside with a reactive derivative derived from a carboxylic acid represented by the formula (IV):

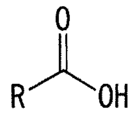

(IV)

wherein R represents optionally substituted lower $C_{1-6}$ or optionally substituted aryl,
using an acid halide represented by the formula (V):

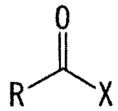

(V)

wherein R represents optionally substituted $C_{1-6}$ alkyl or optionally substituted aryl, and X represents a halogen atom, as the reactive derivative derived from a carboxylic acid represented by the formula (IV) in the presence of a catalytic amount of a Lewis acidic metal halide selected from the group consisting of zinc bromide, cobalt bromide and bismuth bromide.

According to this condition, an acyl group R-C(=O)- of the acid halide (V) is introduced into the hydroxy groups of sugar, and the halogen atom X of the acid halide is further introduced into the anomeric carbon of sugar. The metal halide to be used in this condition acts as a Lewis acid, and shows sufficient effect with a catalytic amount.

Disclosed for reference is a method for producing an α-halo-tetraacyl-glucose represented by the formula (III):

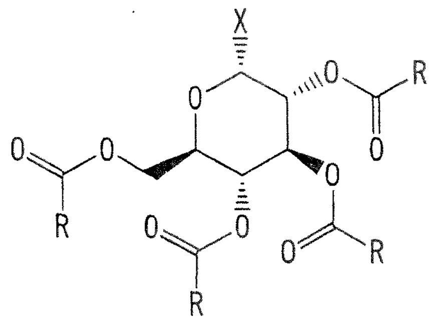

(III)

wherein R represents an optionally substituted lower alkyl or optionally substituted aryl, and X represents a halogen atom, which comprises reacting D-glucoseor lower alkyl D-glucoside with a reactive derivative derived from a carboxylic acid represented by the formula (IV):

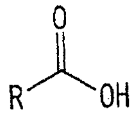

(IV)

wherein R represents optionally substituted lower alkyl or optionally substituted aryl, in the presence of a metal halide represented by the formula: MX

wherein M represents an alkali metal and X represents a halogen atom, a Lewis acid catalyst and a phase-transfer catalyst.

According to this condition, an acyl group R-C(=O)- of the reactive derivative derived from the carboxylic acid (IV) can be introduced into the hydroxy groups of sugar, and the halogen atom X of the metal halide MX can be introduced into the anomeric carbon of sugar, so that a combination of the acyl group R-C(=O)- and the halogen atom X to be introduced can be optionally selected.

In the present invention, the lower alkyl in "optionally substituted lower alkyl" represented by R is a linear and branched lower alkyl having 1 to 6 carbon atoms, and a lower alkyl having 1 to 4 carbon atoms is preferred. More specifically, methyl, ethyl, i-propyl and t-butyl may be mentioned.

The substituent in "optionally substituted lower alkyl" represented by R may be mentioned the same or different 1 to 3 groups selected from a halogen atom (for example, fluorine atom, chlorine atom, etc.), an alkoxy group (for example, methoxy, etc.), an aryloxy group (for example, phenoxy, etc.), etc.

"An optionally substituted lower alkyl" represented by R may be mentioned methyl, chloromethyl, dichloromethyl, trichloromethyl, fluoromethyl, difluoromethyl, trifluoromethyl, methoxymethyl, phenoxymethyl, t-butyl, etc., and methyl and t-butyl are preferred. Among others, t-butyl is preferred.

The aryl in "an optionally substituted aryl" represented by R may be mentioned phenyl, naphthyl, etc., and phenyl is preferred.

The substituent in "an optionally substituted aryl" represented by R may be mentioned the same or different 1 to 3 groups selected from a halogen atom (for example, fluorine atom, chlorine atom, bromine atom, etc.), a hydroxy group, a lower alkyl group (for example, methyl group, etc.), a lower alkoxy group, an aryl group (for example, phenyl, etc.), etc.

"An optionally substituted aryl" represented by R is preferably phenyl.

"An optionally substituted lower alkyl or an optionally substituted aryl" represented by R is preferably methyl, chloromethyl, dichloromethyl, trichloromethyl, fluoromethyl, difluoromethyl, trifluoromethyl, methoxymethyl, phenoxymethyl, t-butyl, phenyl, etc., particularly methyl, t-butyl and phenyl are preferred. Among others, t-butyl is particularly suitable.

The reference reaction of D-glucose or lower alkyl D-glucoside with a reactive derivative derived from a carboxylic acid (IV) and a metal halide can be carried out in a suitable solvent or in the absence of a solvent as follows.

An amount of the reactive derivative derived from the carboxylic acid (IV) may be, for example, 1 to 2 equivalents to a hydroxy group of D-glucose or lower alkyl D-glucoside, and it is suitable to use 5.0-10.0 mol of the reactive derivative derived from a carboxylic acid (IV), preferably 6.5-8.0 mol of the same based on 1 mol of D-glucose or lower alkyl D-glucoside.

When the reference reaction is carried out in the presence of the metal halide MX, the Lewis acid catalyst and the phase-transfer catalyst, the acyl groups R-C(=O)- of the reactive derivative derived from the carboxylic acid (IV) can be introduced into the hydroxy groups of sugar, and the halogen atom X of the metal halide MX can be introduced into an anomeric carbon of sugar. In the reference reaction, M of the metal halide MX is suitably lithium, sodium, etc., and X is preferably chlorine atom, bromine atom, etc. Among others, suitable example of the metal halide MX is lithium

bromide, sodium bromide, etc., and sodium bromide is particularly preferred. A suitable amount of the metal halide MX to be used is generally 5 to 10 mol, preferably 8 mol, per 1 mol of D-glucose or lower alkyl D-glucoside.

In the reference reaction, the Lewis acid suitably used is a metal halide, a metal triflate, silyl triflate, etc., and of these, a metal halide is preferred. The metal halide to be used may include zinc halide (for example, zinc chloride, zinc bromide, etc.), cobalt halide (for example, cobalt chloride, cobalt bromide, etc.), bismuth halide (for example, bismuth chloride, bismuth bromide, etc.), iron halide (for example, iron chloride, iron bromide, etc.), titanium halide (for example, titanium chloride, titanium bromide, etc.), or aluminum halide (for example, aluminum chloride, aluminum bromide), etc., and preferably, zinc chloride, zinc bromide, cobalt chloride, cobalt bromide, bismuth chloride, bismuth bromide, etc., are suitably used. Of these, zinc chloride, zinc bromide, cobalt bromide, bismuth bromide, etc., are preferred, and zinc bromide, cobalt bromide and bismuth bromide are particularly preferred. Among others, zinc bromide is suitably used. The Lewis acid is generally used in an amount of 0.1 to 1 mol, preferably 0.2 mol, per 1 mol of D-glucose or lower alkyl D-glucoside.

In the reference reaction, the phase-transfer catalyst suitably used is a crown ether, a quaternary ammonium salt, etc., and of these, crown ether is preferably used. In particular, 12-crown-4 and 15-crown-5 are preferred, and a combination of lithium halide and 12-crown-4, and a combination of sodium halide and 15-crown-5, etc., are particularly suitable. Among others, a combination of sodium halide and 15-crown-5 is particularly preferred. A suitable amount of the phase-transfer catalyst to be used is generally 0.1 to 1 mol, preferably 0.2 mol, per 1 mol of D-glucose or lower alkyl D-glucoside.

In the reference reaction, R of the reactive derivative derived from the carboxylic acid (IV) is suitably an optionally substituted methyl, t-butyl, an optionally substituted phenyl, etc., more specifically, there may be mentioned methyl, chloromethyl, dichloromethyl, trichloromethyl, fluoromethyl, difluoromethyl, trifluoromethyl, methoxymethyl, phenoxymethyl, t-butyl, phenyl, etc. Of these, methyl, t-butyl, phenyl, etc., are particularly preferred, and among others, t-butyl is suitable. The reactive derivative derived from the carboxylic acid (IV) is preferably an acid halide, and acid chloride, acid bromide, etc., are suitable. In particular, R of the acid halide derived from a carboxylic acid (IV) is suitably an optionally substituted methyl, t-butyl, an optionally substituted phenyl, etc., and among others, t-butyl is suitable. The acid halide derived from the carboxylic acid (IV) is preferably pivaloyl chloride and pivaloyl bromide. Among others, pivaloyl chloride is preferred.

In the reference reaction, preferred conditions include those in which the metal halide MX is lithium halide or sodium halide, the Lewis acid catalyst is a metal halide (preferably, zinc halide, cobalt halide, bismuth halide, iron halide, titanium halide or aluminum halide), and the phase-transfer catalyst is crown ether (preferably, 12-crown-4 or 15-crown-5). Of these, the condition in which the reactive derivative derived from the carboxylic acid (IV) is an acid halide and R of the acid halide is optionally substituted methyl, t-butyl or optionally substituted phenyl (preferably, t-butyl) is suitable.

In the reference reaction, preferred conditions include those in which R of the acid halide derived from the carboxylic acid (IV) is t-butyl and X of the metal halide MX is chlorine atom or bromine atom.

In the reference reaction, other preferred conditions include those in which the reactive derivative derived from the carboxylic acid (IV) is an acid halide, R of the acid halide is t-butyl, the metal halide MX is sodium halide (preferably, sodium chloride or sodium bromide and sodium bromide is more preferred), and the phase-transfer catalyst is 15-crown-5.

In the reference reaction, further preferred conditions include those in which the reactive derivative derived from the carboxylic acid (IV) is an acid halide, R of the acid halide is t-butyl, the metal halide MX is lithium halide (preferably, lithium chloride or lithium bromide and lithium bromide is more preferred), and the phase-transfer catalyst is 12-crown-4.

Moreover, in the reference reaction, still more preferred conditions include those in which the metal halide MX is lithium bromide or sodium bromide, the Lewis acid catalyst is a metal bromide (preferably, zinc bromide, cobalt bromide or bismuth bromide), and the phase-transfer catalyst is 12-crown-4 or 15-crown-5. Of these, conditions in which the reactive derivative derived from the carboxylic acid (IV) is an acid halide and R of the acid halide is t-butyl are preferable.

Furthermore, in the reference reaction, still further preferred conditions include those in which R is t-butyl, the metal halide MX is sodium bromide, the Lewis acid catalyst is a metal bromide (preferably, zinc bromide, cobalt bromide or bismuth bromide), and the phase-transfer catalyst is 15-crown-5.

Among others, in the reference reaction, most preferred conditions include those in which the reactive derivative derived from the carboxylic acid (IV) is pivaloyl chloride, the metal halide MX is sodium bromide, the Lewis acid catalyst is zinc bromide, and the phase-transfer catalyst is 15-crown-5.

In the present invention, when the reaction is carried out by using D-glucose or lower alkyl D-glucoside with the acid halide (V) as the reactive derivative derived from the carboxylic acid (IV), in the presence of a catalytic amount of the Lewis acidic metal halide, then, the acyl groups R-C(=O)- of the acid halide can be introduced into the hydroxy groups of sugar, and the halogen atom X of the acid halide can be introduced into the anomeric carbon of sugar. R of the acid halide (V) is suitably an optionally substituted methyl (for example, chloromethyl, etc.), t-butyl, an optionally substituted phenyl, etc., and among others, particularly preferred are methyl, chloromethyl, dichloromethyl, trichloromethyl, fluoromethyl, difluoromethyl, trifluoromethyl, methoxymethyl, phenoxymethyl, t-butyl, phenyl, etc. Of these, methyl,

t-butyl, phenyl, etc. are particularly preferred, and among others, t-butyl is preferred. X of the acid halide (V) is preferably a chlorine atom or a bromine atom, and among others, the acid halide where X is a bromine atom is suitable. The acid halide (V) is suitably pivaloyl chloride or pivaloyl bromide, and pivaloyl bromide is particularly preferred.

A disclosed Lewis acidic metal halide may include a zinc halide (for example, zinc chloride, zinc bromide, etc.), a cobalt halide (for example, cobalt chloride, cobalt bromide, etc.), a bismuth halide (for example, bismuth chloride, bismuth bromide, etc.), an iron halide (for example, iron chloride, iron bromide, etc.), a titanium halide (for example, titanium chloride, titanium bromide, etc.), or an aluminum halide (for example, aluminum chloride, aluminum bromide), etc., and for example, zinc halide. For example, zinc chloride, zinc bromide, cobalt chloride, cobalt bromide, bismuth chloride, bismuth bromide, etc., are suitably used. Among others, zinc chloride, zinc bromide, cobalt bromide, bismuth bromide, etc. are disclosed. Zinc bromide, cobalt bromide and bismuth bromide are used in the present invention. Also, zinc chloride (reference) and zinc bromide (invention) are

suitably used, and zinc bromide is particularly preferred. The Lewis acidic metal halide is generally used in an amount of 0.1 to 1 mol, preferably 0.2 mol, per 1 mol of D-glucose or lower alkyl D-glucoside. The Lewis acidic metal halide is preferably a metal salt having the same halogen ion for a counter ion as the halogen atom of the acid halide (V) to be used.

In the present reaction, preferred conditions include those in which R of the acid halide (V) is t-butyl, and the Lewis acidic metal halide to be used in a catalytic amount is zinc chloride (reference) and zinc bromide (invention).

In the present reaction, it is also preferred that the conditions include those in which the acid halide (V) is pivaloyl bromide, and the Lewis acidic metal halide to be used in a catalytic amount is zinc bromide, cobalt bromide or bismuth bromide.

Among others, in the present reaction, more preferred conditions include those in which the acid halide (V) is pivaloyl bromide, and the Lewis acidic metal halide to be used in a catalytic amount is zinc bromide.

In the reaction according to the present invention, when an acid halide having both of an acyl group to be introduced into the hydroxy groups of D-glucose and a halogen atom to be introduced into the anomeric carbon of D-glucose is easily available, then, the acid halide may be reacted with D-glucose or lower alkyl D-glucoside in the presence of a catalytic amount of the Lewis acidic metal halide.

In the reaction according to the present invention, when an acyl group to be introduced into the hydroxy groups of D-glucose and a halogen atom to be introduced into the anomeric carbon of D-glucose are to be optionally combined, a reactive derivative derived from the carboxylic acid (IV) having a desired acyl group and a metal halide MX having a desired a halogen atom X are reacted with D-glucose or lower alkyl D-glucoside in the presence of a Lewis acid catalyst and a phase-transfer catalyst.

The solvent is not particularly limited so long as it does no exert any harmful effect to the reaction, and there may be optionally used, for example, acetonitrile, ethers (for example, tetrahydrofuran, diethyl ether, dioxane, 1,2-dimethoxyethane), hydrocarbons (for example, toluene, xylene, benzene), halogenated hydrocarbons (for example, methylene chloride, dichloroethane, chloroform, chlorobenzene), or a mixed solvent of the above, preferably halogenated hydrocarbons, and methylene chloride is particularly preferred.

The reaction temperature can be in general optionally selected from 0 to 110°C, and suitably from room temperature to 40°C.

The reaction time can be adjusted depending on the reaction condition.

The carboxylic acid (IV) is commercially available or can be prepared easily according to the method conventionally known to those skilled in the art. The reactive derivative derived from the carboxylic acid (IV) can be also prepared according to the conventional method.

The compound thus obtained can be isolated in a free form or in a form of a salt thereof, and purified. The salt can be prepared by subjecting the obtained compound to the salt formulating method generally used. Isolation and purification can be carried out according to the conventional and commonly used methods in the organic synthesis chemistry such as extraction, concentration, crystallization, filtration, recrystallization, various kinds of chromatographies, etc.

The $\alpha$-halo-tetraacyl-glucose (III) obtained as mentioned above can be converted into canagliflozin represented by the formula (1):

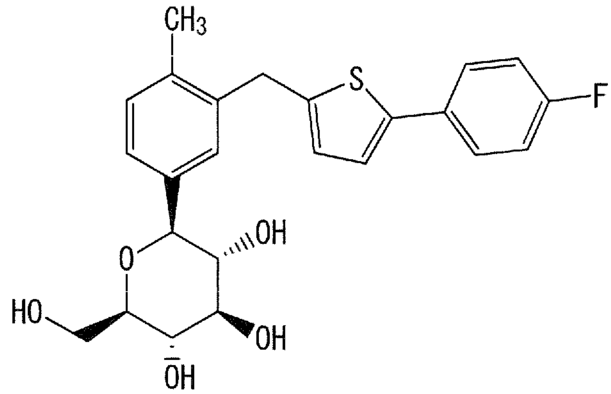

(I)

or a pharmaceutically acceptable salt thereof by subjecting to a conventional method. The conventional method may be mentioned, for example, a method disclosed in Patent Literature 2 (WO 2011/047113A). That is, the α-halo-tetraacyl-glucose represented by the formula (III) prepared as mentioned above and an iodated aglycone (VI) are subjected to C-glycosylation reaction, and then, the acyl groups of the hydroxy groups of the resulting Compound (II) are removed to give the objective product (Canagliflozin) represented by the formula (I).

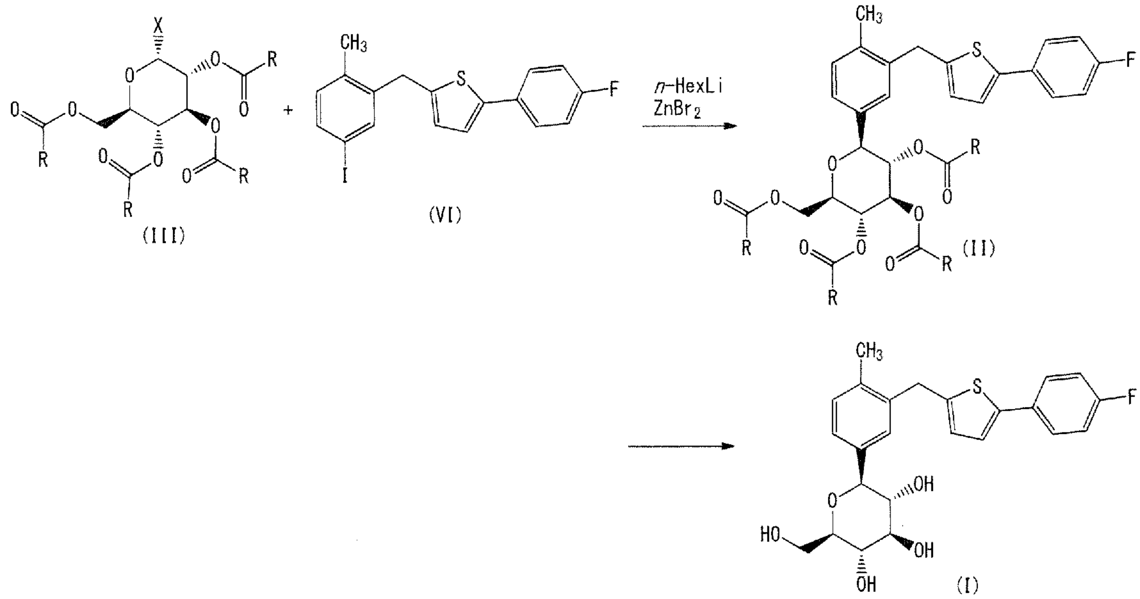


(III) (VI) (II) (I)

That is, the compound represented by the formula (VI):

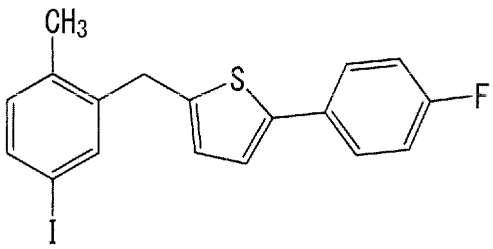

(VI)

is subjected to a halogen-metal exchange reaction by using alkyl lithium (for example, n-hexyl lithium, etc.,) to convert it to a compound represented by the formula (VII):

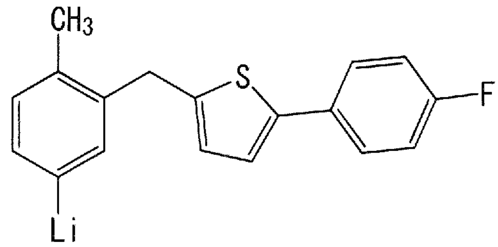

(VII)

then, zinc salt (for example, zinc bromide) is acted thereon to carry out a transmetallation reaction to obtain a compound represented by the formula (VIII):

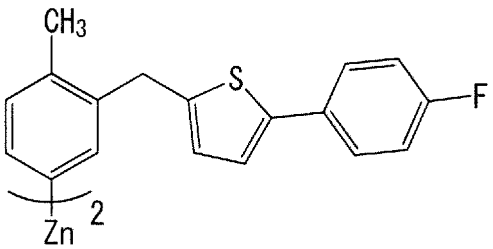

(VIII)

and the resulting compound represented by the formula (VIII) and an $\alpha$-halo-tetraacyl-glucose (III) are subjected to coupling reaction to obtain a compound represented by the formula (II):

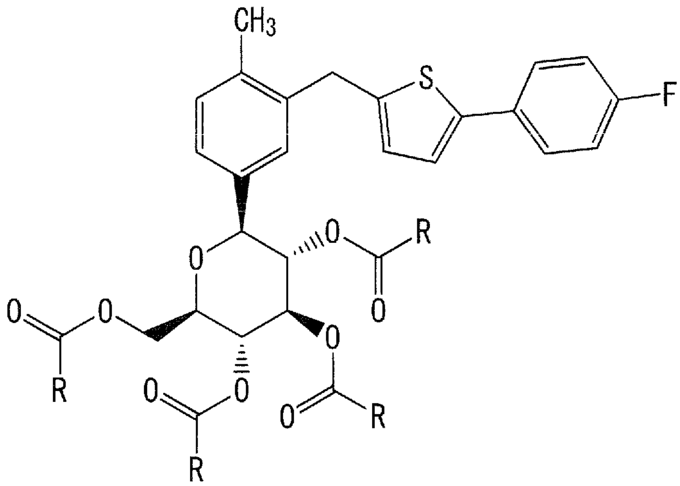

(II)

wherein the symbols have the same meanings as defined above. From the compound represented by the formula (II), the acyl groups R-C(=O)- are removed by using, for example, a base, optionally followed by converting to a pharmaceutically acceptable salt thereof, to afford Canagliflozin represented by the formula (I):

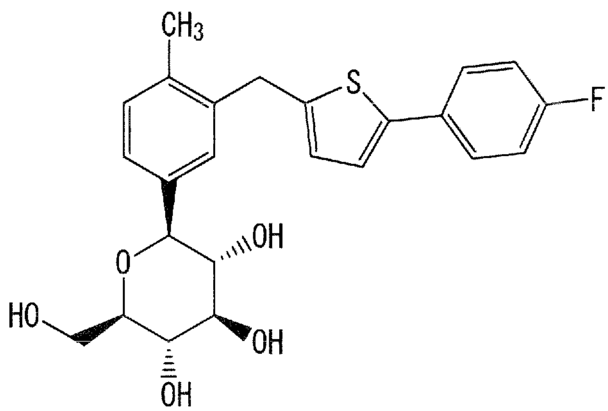

(I)

or a pharmaceutically acceptable salt thereof.

In addition, Canagliflozin can be also prepared, for example, according to the method described in Patent Literature 3. That is, the compound represented by the formula (VI):

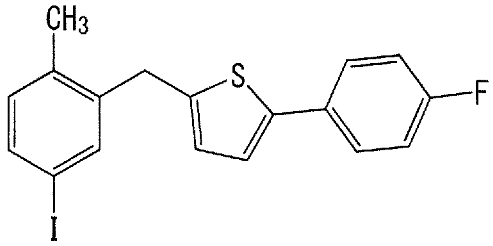

(VI)

is treated with an alkyl lithium, for example, n-hexyl lithium, etc., and zinc salt, for example zinc bromide, etc., in a hydrocarbon solvent, for example, toluene, etc., to afford a compound represented by the formula (VII):

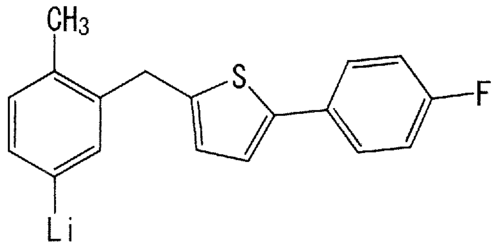

(VII)

then, followed by adding a ether solvent, for example, dibutyl ether, etc., to the reaction mixture to afford a compound represented by the formula (VIII):

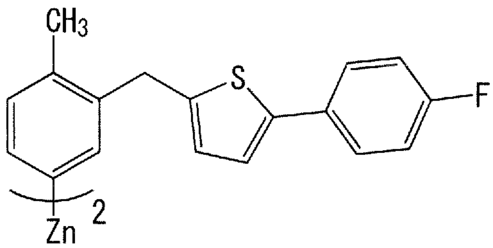

(VIII)

and the resulting compound represented by the formula (VIII) and an α-halo-tetraacyl-glucose (III) are subjected to coupling reaction to obtain a compound represented by the formula (II):

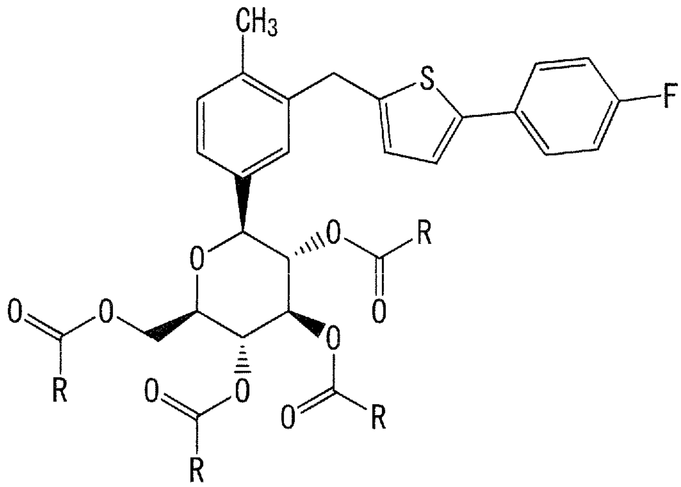

(II)

wherein the symbols have the same meanings as defined above. From the compound represented by the formula (II),

the acyl groups R-C(=O)- are removed by using, for example, a base, optionally followed by converting to a pharmaceutically acceptable salt thereof, for example, hydrate, hemihydrate ,etc., to afford Canagliflozin represented by the formula (I):

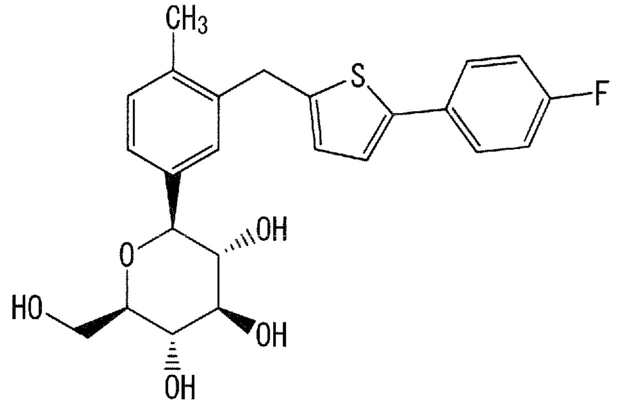

(I)

or a pharmaceutically acceptable salt thereof.

In addition, Canagliflozin can be also prepared, for example, according to the method described in Non-Patent Literature 1 (Organic Letters, 2012, vol. 14, No. 6, pp. 1480-1483). That is, Compound (VI) is subjected to a halogen-metal exchange reaction by using, for example, n-butyl lithium, to convert it into Compound (VII), then, for example, zinc bromide lithium bromide is acted thereon to carry out a transmetallation reaction to obtain Compound (VIII), and the obtained Compound (VIII) and an $\alpha$-halo-tetraacyl-glucose (III) are subjected to coupling reaction to obtain Compound (II). The acyl groups R-C(=O)- of obtained Compound (II) are removed by using, for example, a base to obtain Canagliflozin (I) or a pharmaceutically acceptable salt thereof.

Furthermore, Dapagliflozin can be also prepared, for example, according to the method described in Non-Patent Literature 1 (Organic Letters, 2012, vol. 14, No. 6, pp. 1480-1483). That is, the compound of the following formula:

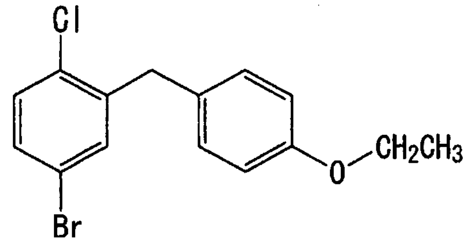

is reacted with, for example, lithium di-n-butyl n-hexyl magneciate, and the resulting mixture is treated with zinc bromide lithium bromide, and then the obtained compound and an $\alpha$-halo-tetraacyl-glucose (III) are subjected to coupling reaction and acyl groups R-C(=O)- are removed from the obtained compound by using, for example, a base to obtain Dapagliflozin.

According to the present invention, both of D-glucose and $C_{1-6}$ alkyl D-glucoside can be suitably used as a starting material for the production of an $\alpha$-halo-tetraacyl-glucose (III).

And, when $C_{1-6}$ alkyl D-glucoside (for example, methyl D-glucoside or ethyl D-glucoside) is used as the starting material, preparation of an $\alpha$-halo-tetraacyl-glucose (III) can be suitably conducted by using acid halides (for example, pivaloyl bromide) and zinc bromide.

As used herein, the abbreviations "Me" means methyl group, "Et" means ethyl group, "Ph" means phenyl group, "Ac" means acetyl group, and "t-Bu" means tertiary butyl group.

The compound of the formula (I) includes a pharmaceutically acceptable salt thereof, such as an intramolecular salt, a hydrate (including a hemihydrate), a solvate and a crystal polymorphism. The pharmaceutically acceptable salt thereof includes, for example, a salt with an alkali metal such as lithium, sodium and potassium, etc.; a salt with an alkaline earth metal such as calcium and magnesium, etc.; a salt with zinc or aluminum; a salt with an organic base such as ammonium, choline, diethanolamine, lysine, ethylenediamine, t-butylamine, t-octylamine, tris(hydroxymethyl)aminomethane, N-methylglucosamine, triethanolamine and dehydroabiethylamine; a salt with an inorganic acid such as hydrochloric acid, hydrobromic acid, hydroiodic acid, sulfuric acid, nitric acid, phosphoric acid, etc.; a salt with an organic acid such as formic acid, acetic acid, propionic acid, oxalic acid, malonic acid, succinic acid, fumaric acid, maleic acid, lactic acid, malic acid, tartaric acid, citric acid, methanesulfonic acid, ethanesulfonic acid, benzenesulfonic acid, etc.; or a salt with an acidic amino acid such as aspartic acid, glutamic acid, etc.

As used in the specification and the claims, D-glucose includes both α-D-glucose and β-D-glucose, and lower alkyl D-glucoside includes both lower alkyl α-D-glucoside and lower alkyl β-D-glucoside.

EXAMPLES

In the following, the present invention is explained in more detail by referring to Examples, but the present invention is not limited thereto.

Example 1

Preparation of (2R,3R,4S,5R,6R)-2-bromo-6-(acetyloxymethyl)tetrahydro-2H-pyran-3,4,5-triyl triacetate

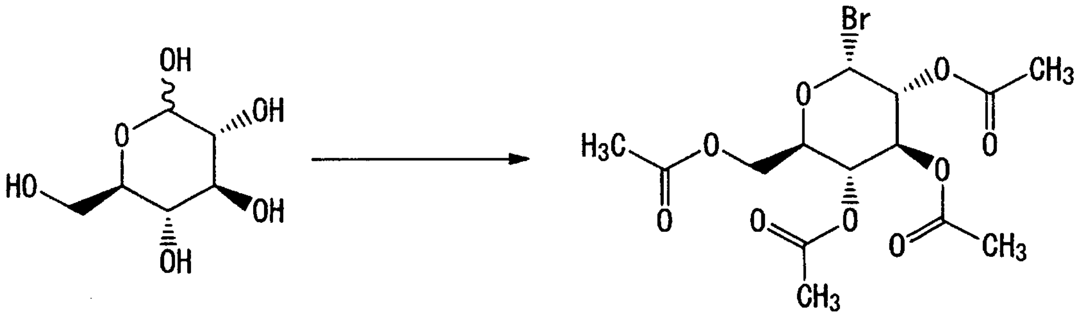

D-glucose (1.00 g, 5.55 mmol) was added to a mixture comprising acetyl bromide (5.52 g, 44.90 mmol), zinc bromide (255.3 mg, 1.13 mmol) and dichloromethane (10 mL), and the resulting mixture was stirred at room temperature for 26 hours. Under ice-cooling, the reaction mixture was distributed and washed with water and ethyl acetate, the organic layer was washed with a 10% aqueous sodium hydrogencarbonate solution, dried over magnesium sulfate, and then, filtered and concentrated. The concentrated residue was recrystallized from isopropyl ether, to obtain the title compound as colorless crystals (1.42 g, Yield: 62%).

Example 2 (reference)

Preparation of (2R,3R,4S,5R,6R)-2-chloro-6-(benzoyloxymethyl)tetrahydro-2H-pyran-3,4,5-triyl tribenzoate

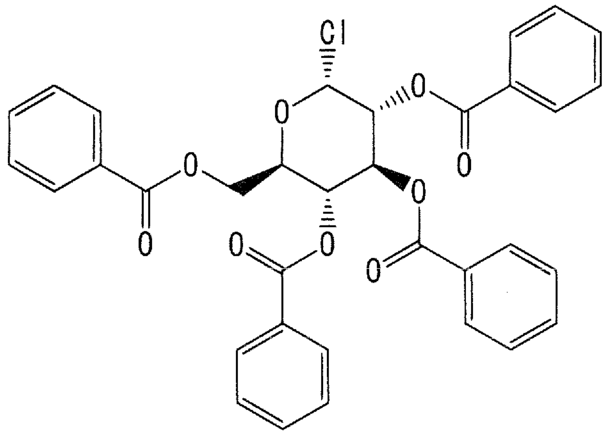

Zinc chloride (164.7 mg, 1.21 mmol) was added to a mixture comprising benzoyl chloride (7.84 g, 55.77 mmol), D-glucose (1.00 g, 5.55 mmol) and dichloromethane (10 mL), and the resulting mixture was stirred under ice-cooling for 18 hours. After raising the temperature of the mixture to room temperature, the mixture was stirred for further 26 hours. After raising the temperature of the mixture to 40°C and stirring for 2 hours, the reaction mixture was washed with water

under ice-cooling. The obtained organic layer was washed twice with a 10% aqueous sodium hydrogencarbonate solution, and dried over magnesium sulfate. After the organic layer was further washed with water, a 10% aqueous sodium hydrogencarbonate solution was added to the same and the resulting mixture was stirred at 40°C for 13.5 hours. The separated organic layer was dried over magnesium sulfate, filtered and then concentrated. The concentrated residue was purified by column chromatography (SH silica gel, hexane/ethyl acetate) to obtain the title compound (2.93 g, Yield: 86%).

Example 3

Preparation of (2R,3R,4S,5R,6R)-2-bromo-6-(benzoyloxymethyl)tetrahydro-2H-pyran-3,4,5-triyl tribenzoate

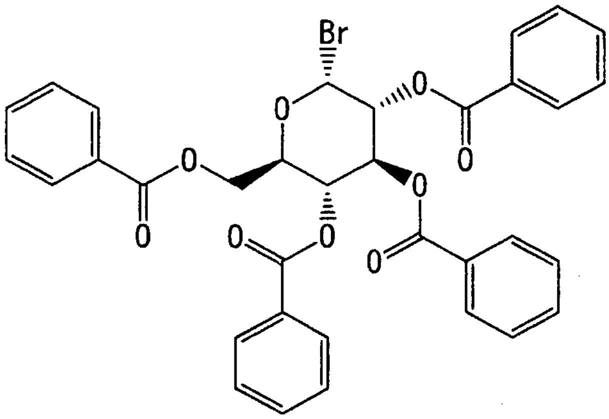

D-glucose (1.01 g, 5.61 mmol) was added to a mixture comprising benzoyl bromide (10.30 g, 55.67 mmol), zinc bromide (252.2 mg, 1.12 mmol) and dichloromethane (10 mL) under ice-cooling, and the resulting mixture was stirred at room temperature for 4 days. Under ice-cooling, the reaction mixture was distributed and washed with water and dichloromethane, and the organic layer was washed twice with a 10% aqueous sodium hydrogencarbonate solution. The obtained organic layer was dried over magnesium sulfate, filtered and then concentrated. The concentrated residue was purified by column chromatography (SH silica gel, hexane/ethyl acetate) to obtain the title compound (2.34 g, Yield: 64%).

Example 4 (reference)

Preparation of (2R,3R,4S,5R,6R)-2-chloro-6-(pivaloyloxymethyl)tetrahydro-2H-pyran-3,4,5-triyl tris(2,2-dimethylpropanoate)

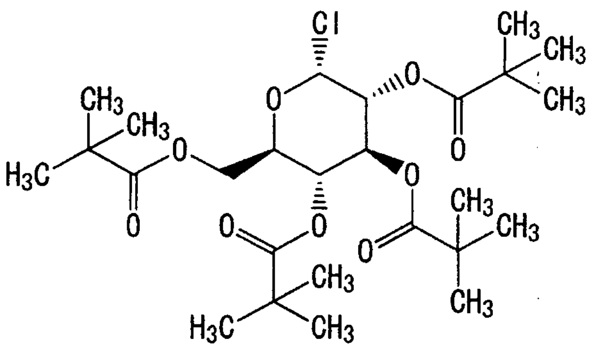

D-glucose (1.01 g, 5.61 mmol) was added to a mixture comprising pivaloyl chloride (5.32 g, 44.12 mmol), zinc chloride (150.8 mg, 1.11 mmol) and dichloromethane (10 mL), and the resulting mixture was stirred at room temperature

for 19 hours. The reaction mixture was washed with water, and the organic layer was dried over magnesium sulfate, filtered and then concentrated. The concentrated residue was purified by silica gel column chromatography (silica gel, hexane/ethyl acetate) to obtain the title compound as crystals (2.03 g, Yield: 68%).

Example 5

Preparation of (2R,3R,4S,5R,6R)-2-bromo-6-(pivaloyloxymethyl)tetrahydro-2H-pyran-3,4,5-triyl tris(2,2-dimethylpropanoate)

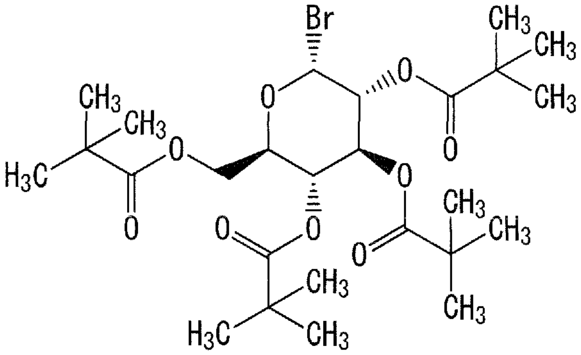

A mixture comprising pivaloyl chloride (5.52 g, 45.78 mmol), trimethylsilane bromide (6.12 g, 39.98 mmol) and dichloromethane (10 mL) was stirred under ice-cooling for 2 hours, then, zinc bromide (II) (257.8 mg, 1.14 mmol) and D-glucose (1 g, 5.55 mmol) were added to the mixture, and a temperature of the resulting mixture was raised to room temperature and stirring was further continued. After 18.5 hours from the start of the reaction, completion of the reaction was confirmed. The reaction mixture was washed with water under room temperature, and the organic layer was sampled to prepare a sample solution and it was analyzed by HPLC.
HPLC measurement conditions
Column: Cadenza CD-C18 (25 cm x 4.6 mm, 3 μm)
Mobile phase: Mobile phase A: purified water, Mobile phase B: acetonitrile
Column temperature: 40°C
Flow rate: 1.0 ml/min
Detection wavelength: 210 nm
Preparation of sample solution: Sample (about 280 mg) was measured in a volumetric flask, and diluted with a 90% acetonitrile solution.
Retention time of the title compound was 27 minutes. The yield was calculated by the following equation.

$$\text{Yield (\%)}=[\text{Area of } \alpha\text{-halo-tetraacyl compound}]/A/B \times C/[\text{Molecular weight of } \alpha\text{-halo-tetraacyl compound}]/[\text{Mole number of D-glucose used}] \times 100$$

A: Ratio (area/(g/L)) of an HPLC area to a concentration of the solution in an α-halo-tetraacyl compound-standard solution
B: Concentration (g/L) of the sample solution
C: Whole amount (g) of the reaction solution

The calculated yield was 67%.

Example 6

Preparation of (2R,3R,4S,5R,6R)-2-bromo-6-(pivaloyloxymethyl)tetrahydro-2H-pyran-3,4,5-triyl tris(2,2-dimethylpropanoate)

A mixture comprising pivaloyl chloride (5.21 g, 43.21 mmol), trimethylsilane bromide (6.9 g, 45.07 mmol) and dichloromethane (10 mL) was stirred for 2 hours, then, bismuth(III) bromide (519.6 mg, 1.16 mmol) and D-glucose (1 g,

5.55 mmol) were added to the mixture, and the resulting mixture was further stirred. After 17.5 hours from the starting of the reaction, completion of the reaction was confirmed. The reaction mixture was washed with water under ice-cooling, and the organic layer was sampled. HPLC analysis was carried out in the same manner as in Example 5 to carry out identification and determination of quantity of the objective product. The calculated yield was 68%.

Example 7

Preparation of (2R,3R,4S,5R,6R)-2-bromo-6-(pivaloyloxymethyl)tetrahydro-2H-pyran-3,4,5-triyl tris(2,2-dimethylpropanoate)

A mixture comprising pivaloyl chloride (5.45 g, 45.19 mmol), trimethylsilane bromide (6.9 g, 45.07 mmol) and dichloromethane (10 mL) was stirred for 2 hours, cobalt(II) bromide (250.4 mg, 1.14 mmol) and D-glucose (1 g, 5.55 mmol) were added to the mixture, and the resulting mixture was further stirred. After 40 hours from the start of the reaction, completion of the reaction was confirmed. The reaction mixture was washed under ice-cooling with water and then with a 10% aqueous sodium hydrogencarbonate solution, and the organic layer was sampled. HPLC analysis was carried out in the same manner as in Example 5 to carry out identification and determination of quantity of the objective product. The calculated yield was 49%.

Example 8

Preparation of (2R,3R,4S,5R,6R)-2-bromo-6-(pivaloyloxymethyl)tetrahydro-2H-pyran-3,4,5-triyl tris(2,2-dimethylpropanoate)

Thionyl bromide (9.39 g, 45.17 mmol) was added dropwise to a mixture comprising pivalic acid (4.53 g, 44.36 mmol), N-methylpyrrolidone (53.5 μL, 0.55 mmol) and dichloromethane (10 mL) at 0°C under stirring over 30 minutes. The resulting mixture was stirred at room temperature for 2 hours, then, zinc bromide (252.1 mg, 1.12 mmol) and D-glucose (lg, 5.55 mmol) were added to the mixture, and the resulting mixture was further stirred. After 22.5 hours from starting the reaction, completion of the reaction was confirmed. The reaction mixture was washed with water and then with a 10% aqueous sodium hydrogencarbonate solution, and the obtained organic layer was sampled. HPLC analysis was carried out in the same manner as in Example 5 to carry out identification and determination of quantity of the objective product. The calculated yield was 49%.

Example 9

Preparation of (2R,3R,4S,5R,6R)-2-bromo-6-(pivaloyloxymethyl)tetrahydro-2H-pyran-3,4,5-triyl tris(2,2-dimethylpropanoate)

A mixture comprising pivaloyl chloride (4.39 g, 36.1 mmol), sodium bromide (5.00 g, 45.78 mmol), 15-crown-5 (132.3mg, 0.56 mmol) and dichloromethane (10 mL) was stirred at 40°C for 3.5 hours, and then, zinc (II) bromide (265.3 mg, 1.14 mmol) and D-glucose (1 g, 5.55 mmol) were added to the mixture. The resulting mixture was further stirred at 40°C. After 20 hours from the start of the reaction, the reaction mixture was washed at room temperature with water and then with a 10% aqueous sodium hydrogencarbonate solution, and the organic layer was sampled. HPLC analysis was carried out in the same manner as in Example 5 to carry out identification and determination of quantity of the objective product. The calculated yield was 72%.

Example 10

Preparation of (2R,3R,4S,5R,6R)-2-bromo-6-(pivaloyloxymethyl)tetrahydro-2H-pyran-3,4,5-triyl tris(2,2-dimethylpropanoate)

A mixture of pivaloyl chloride (40.36 g, 334.74 mmol), lithium bromide (34.89 g, 401.69 mmol) and dichloromethane (120 mL) was stirred for 5 hours under nitrogen atmosphere and filtered. The residue was washed with dichloromethane (80 mL) to obtain the solution of pivaloyl bromide in dichloromethane.

To the mixture of zinc bromide (2.32 g, 10.3 mmol) and methyl α-D-glucoside (10.0g, 51.5 mmol) was added the solution of pivaloyl bromide obtained above, and the mixture was stirred at 40°C for 5 hours. The mixture was cooled to room temperature, washed with water and then with a 10% aqueous sodium hydrogencarbonate solution, and the organic layer was concentrated. The residue was recrystallized from acetone-water to obtain the titled compound as colorless crystal (24.8 g, 83 %yield).

Example 11

Preparation of (2R,3R,4S,5R,6R)-2-bromo-6-(pivaloyloxymethyl)tetrahydro-2H-pyran-3,4,5-triyl tris(2,2-dimethylpropanoate)

A mixture comprising pivaloyl chloride (8.27 g, 68.6 mmol), sodium bromide (8.71 g, 84.7 mmol), 15-crown-5 (0.227 g, 1.03 mmol) and dichloromethane (40 mL) was stirred at 40°C for 1 hour, and then, zinc (II) bromide (0.47 g, 2.09 mmol) and methyl α-D-glucoside (2 g, 10.3 mmol) were added to the mixture. The resulting mixture was further stirred at 40°C. After 23 hours from the start of the reaction, the reaction mixture was washed at room temperature with water and then with a 10% aqueous sodium hydrogencarbonate solution, and the organic layer was sampled. HPLC analysis was carried out in the same manner as in Example 5 to carry out identification and determination of quantity of the objective product. The calculated yield was 65%.

Example 12

Preparation of (2R,3R,4S,5R,6R)-2-bromo-6-(pivaloyloxymethyl)tetrahydro-2H-pyran-3,4,5-triyl tris(2,2-dimethylpropanoate)

A mixture comprising sodium bromide (4.11 g, 39.9 mmol), 15-crown-5 (95 μL, 0.48 mmol) and dichloromethane (20 mL) was stirred at 40°C for 2.5 hours, and then, pivaloyl chloride (3.80 g, 31.5 mmol), zinc (II) bromide (236.6 mg, 1.05 mmol) and ethyl α-D-glucoside (1.01 g, 4.85 mmol) were added to the mixture. The resulting mixture was further stirred at 40°C. After 40.5 hours from the start of the reaction, the reaction mixture was washed at room temperature with water and then with a 10% aqueous sodium hydrogencarbonate solution, and the organic layer was sampled. HPLC analysis was carried out in the same manner as in Example 5 to carry out identification and determination of quantity of the objective product. The calculated yield was 58%.

INDUSTRIAL APPLICABILITY

According to the preparation method of the present invention, an α-halo-tetraacyl-glucose useful as a synthetic intermediate, etc., of a medicine can be industrially advantageously and efficiently prepared. In addition, Canagliflozin or a salt thereof which is useful as a medicine, etc., can be industrially advantageously and efficiently prepared.

## Claims

1. A method for producing an α-halo-tetraacyl-glucose represented by the formula (III):

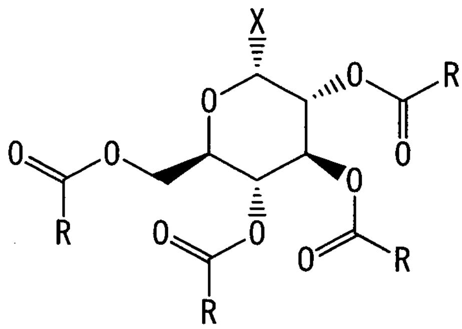

(III)

wherein R represents optionally substituted $C_{1-6}$ alkyl or optionally substituted aryl, and X represents a halogen atom, which comprises reacting D-glucose or $C_{1-6}$ alkyl D-glucoside with an acid halide represented by the formula (V):

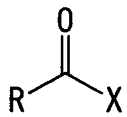

(V)

wherein R represents an optionally substituted $C_{1-6}$ alkyl or an optionally substituted aryl, and X represents a halogen

atom,
in the presence of a catalytic amount of a Lewis acidic metal halide selected from the group consisting of zinc bromide, cobalt bromide and bismuth bromide.

**2.** The method according to claim 1, wherein the Lewis acidic metal halide is used in an amount of 0.1 to 1 mol per 1 mol of D-glucose or $C_{1-6}$ alkyl D-glucoside.

**3.** The method according to claim 1, wherein the Lewis acidic metal halide is used in an amount of 0.1 to 0.2 mol per 1 mol of D-glucose or $C_{1-6}$ alkyl D-glucoside.

**4.** The method according to claim 1, wherein the method comprises reacting $C_{1-6}$ alkyl D-glucoside with an acid halide represented by the formula (V):

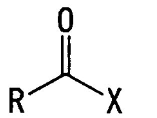

(V)

wherein R represents an optionally substituted $C_{1-6}$ alkyl or an optionally substituted aryl, and X represents a halogen atom,
in the presence of a catalytic amount of a Lewis acidic metal halide selected from the group consisting of zinc bromide, cobalt bromide and bismuth bromide.

**5.** The method according to claim 1, wherein the method comprises reacting $C_{1-6}$ alkyl D-glucoside with an acid halide represented by the formula (V):

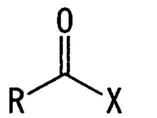

(V)

wherein R represents an optionally substituted $C_{1-6}$ alkyl or an optionally substituted aryl, and X represents a halogen atom,
in the presence of a catalytic amount of a Lewis acidic metal halide selected from the group consisting of zinc bromide, cobalt bromide and bismuth bromide,
wherein the Lewis acidic metal halide is used in an amount of 0.1 to 1 mol per 1 mol of $C_{1-6}$ alkyl D-glucoside.

**6.** The method according to claim 1, wherein the method comprises reacting $C_{1-6}$ alkyl D-glucoside with an acid halide represented by the formula (V):

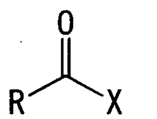

(V)

wherein R represents an optionally substituted $C_{1-6}$ alkyl or an optionally substituted aryl, and X represents a halogen atom
in the presence of a catalytic amount of a Lewis acidic metal halide selected from the group consisting of zinc bromide, cobalt bromide and bismuth bromide,
wherein the Lewis acidic metal halide is used in an amount of 0.1 to 0.2 mol per 1 mol of $C_{1-6}$ alkyl D-glucoside.

**7.** The method according to any one of claims 1 to 6, wherein R of the acid halide is an optionally substituted methyl, t-butyl or an optionally substituted phenyl.

**8.** The method according to claim 7, wherein R is t-butyl.

**9.** The method according to any one of claims 1 to 8, wherein X is a chlorine atom or a bromine atom.

10. The method according to any one of claims 1 to 9, wherein the Lewis acidic metal halide is zinc bromide.

11. The method according to any one of claims 1 to 10, wherein the acid halide represented by the formula (V) is pivaloyl bromide.

**Patentansprüche**

1. Ein Verfahren zur Herstellung einer α-Halo-Tetraacyl-Glukose, dargestellt durch die Formel (III):

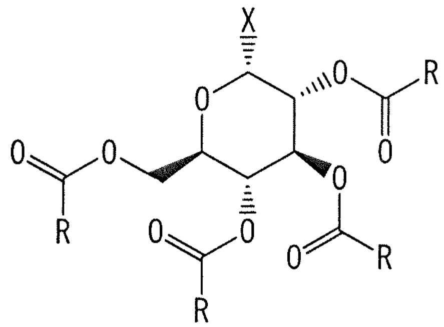

(III)

wobei R gegebenenfalls substituiertes $C_{1-6}$-Alkyl oder gegebenenfalls substituiertes Aryl darstellt, und X ein Halogenatom darstellt,
welches das Umsetzen von D-Glukose oder $C_{1-6}$-Alkyl-D-Glukosid mit einem Säurehalogenid, dargestellt durch die Formel (V), umfasst:

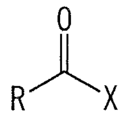

(V)

wobei R ein gegebenenfalls substituiertes $C_{1-6}$-Alkyl oder ein gegebenenfalls substituiertes Aryl darstellt, und X ein Halogenatom darstellt,
in Gegenwart einer katalytischen Menge eines Lewis sauren Metallhalogenids, ausgewählt aus der Gruppe bestehend aus Zinkbromid, Kobaltbromid und Bismuthbromid.

2. Das Verfahren gemäß Anspruch 1, wobei das Lewis saure Metallhalogenid in einer Menge von 0,1 bis 1 Mol pro 1 Mol D-Glukose oder $C_{1-6}$-Alkyl-D-Glukosid verwendet wird.

3. Das Verfahren gemäß Anspruch 1, wobei das Lewis saure Metallhalogenid in einer Menge von 0,1 bis 0,2 Mol pro 1 Mol D-Glukose oder $C_{1-6}$-Alkyl-D-Glukosid verwendet wird.

4. Das Verfahren gemäß Anspruch 1, wobei das Verfahren das Umsetzen von $C_{1-6}$-Alkyl-D-Glukosid mit einem Säurehalogenid, dargestellt durch die Formel (V), umfasst:

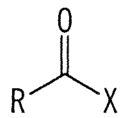

(V)

wobei R ein gegebenenfalls substituiertes $C_{1-6}$-Alkyl oder ein gegebenenfalls substituiertes Aryl darstellt, und X ein Halogenatom darstellt,
in Gegenwart einer katalytischen Menge eines Lewis sauren Metallhalogenids, ausgewählt aus der Gruppe bestehend aus Zinkbromid, Kobaltbromid und Bismuthbromid.

5. Das Verfahren gemäß Anspruch 1, wobei das Verfahren das Umsetzen von $C_{1-6}$-Alkyl-D-Glukosid mit einem Säurehalogenid, dargestellt durch die Formel (V), umfasst:

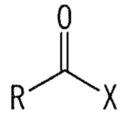

(V)

wobei R ein gegebenenfalls substituiertes $C_{1-6}$-Alkyl oder ein gegebenenfalls substituiertes Aryl darstellt, und X ein Halogenatom darstellt,
in Gegenwart einer katalytischen Menge eines Lewis sauren Metallhalogenids, ausgewählt aus der Gruppe bestehend aus Zinkbromid, Kobaltbromid und Bismuthbromid,
wobei das Lewis saure Metallhalogenid in einer Menge von 0,1 bis 1 Mol pro 1 Mol $C_{1-6}$-Alkyl-D-Glukosid verwendet wird.

**6.** Das Verfahren gemäß Anspruch 1, wobei das Verfahren das Umsetzen von $C_{1-6}$-Alkyl-D-Glukosid mit einem Säurehalogenid, dargestellt durch die Formel (V), umfasst:

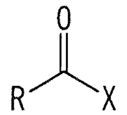

(V)

wobei R ein gegebenenfalls substituiertes $C_{1-6}$-Alkyl oder ein gegebenenfalls substituiertes Aryl darstellt, und X ein Halogenatom darstellt,
in Gegenwart einer katalytischen Menge eines Lewis sauren Metallhalogenids, ausgewählt aus der Gruppe bestehend aus Zinkbromid, Kobaltbromid und Bismuthbromid,
wobei das Lewis saure Metallhalogenid in einer Menge von 0,1 bis 0,2 Mol pro 1 Mol $C_{1-6}$-Alkyl-D-Glukosid verwendet wird.

**7.** Das Verfahren gemäß einem der Ansprüche 1 bis 6, wobei R des Säurehalogenids ein gegebenenfalls substituiertes Methyl, t-Butyl oder ein gegebenenfalls substituiertes Phenyl ist.

**8.** Das Verfahren gemäß Anspruch 7, wobei R für t-Butyl steht.

**9.** Das Verfahren gemäß einem der Ansprüche 1 bis 8, wobei X ein Chloratom oder ein Bromatom ist.

**10.** Das Verfahren gemäß einem der Ansprüche 1 bis 9, wobei das Lewis saure Metallhalogenid Zinkbromid ist.

**11.** Das Verfahren gemäß einem der Ansprüche 1 bis 10, wobei das durch die Formel (V) dargestellte Säurehalogenid Pivaloylbromid ist.

## Revendications

**1.** Procédé de production d'un α-halo-tétraacyl-glucose représenté par la formule (III) :

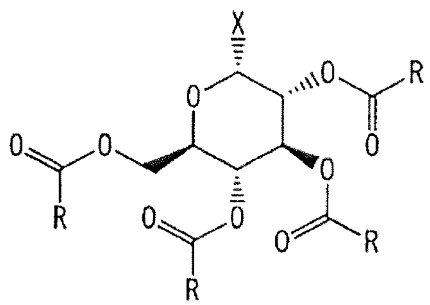

(III)

où R représente un groupe alkyle en $C_{1-6}$ éventuellement substitué ou aryle éventuellement substitué, et X représente un atome d'halogène,
qui comprend la réaction de D-glucose ou D-glucoside d'alkyle en $C_{1-6}$ avec un halogénure d'acide représenté par

la formule (V) :

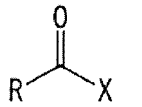 (V)

où R représente un groupe alkyle en $C_{1-6}$ éventuellement substitué ou un groupe aryle éventuellement substitué, et X représente un atome d'halogène,
en présence d'une quantité catalytique d'un halogénure de métal d'acide de Lewis choisi dans le groupe consistant en bromure de zinc, bromure de cobalt et bromure de bismuth.

2. Procédé selon la revendication 1, dans lequel l'halogénure de métal d'acide de Lewis est utilisé dans une quantité de 0,1 à 1 mol pour 1 mole de D-glucose ou D-glucoside d'alkyle en $C_{1-6}$.

3. Procédé selon la revendication 1, dans lequel l'halogénure de métal d'acide de Lewis est utilisé dans une quantité de 0,1 à 0,2 mol pour 1 mole de D-glucose ou D-glucoside d'alkyle en $C_{1-6}$.

4. Procédé selon la revendication 1, dans lequel le procédé comprend la réaction de D-glucoside d'alkyle en $C_{1-6}$ avec un halogénure d'acide représenté par la formule (V) :

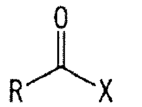 (V)

où R représente un groupe alkyle en $C_{1-6}$ éventuellement substitué ou un groupe aryle éventuellement substitué, et X représente un atome d'halogène,
en présence d'une quantité catalytique d'un halogénure de métal d'acide de Lewis choisi dans le groupe consistant en bromure de zinc, bromure de cobalt et bromure de bismuth.

5. Procédé selon la revendication 1, où le procédé comprend la réaction de D-glucoside d'alkyle en $C_{1-6}$ avec un halogénure d'acide représenté par la formule (V) :

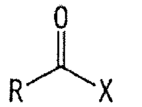 (V)

où R représente un groupe alkyle en $C_{1-6}$ éventuellement substitué ou un groupe aryle éventuellement substitué, et X représente un atome d'halogène,
en présence d'une quantité catalytique d'un halogénure de métal d'acide de Lewis choisi dans le groupe consistant en bromure de zinc, bromure de cobalt et bromure de bismuth,
où l'halogénure de métal d'acide de Lewis est utilisé dans une quantité de 0,1 à 1 mol pour 1 mole de D-glucoside d'alkyle en $C_{1-6}$.

6. Procédé selon la revendication 1, où le procédé comprend la réaction de D-glucoside d'alkyle en $C_{1-6}$ avec un halogénure d'acide représenté par la formule (V) :

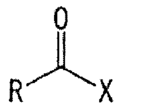 (V)

où R représente un groupe alkyle en $C_{1-6}$ éventuellement substitué ou groupe aryle éventuellement substitué, et X représente un atome d'halogène,
en présence d'une quantité catalytique d'un halogénure de métal d'acide de Lewis choisi dans le groupe consistant en bromure de zinc, bromure de cobalt et bromure de bismuth,

où l'halogénure de métal d'acide de Lewis est utilisé dans une quantité de 0,1 à 0,2 mol pour 1 mole de D-glucoside d'alkyle en $C_{1-6}$.

**7.** Procédé selon l'une quelconque des revendications 1 à 6, où R de l'halogénure d'acide est un groupe méthyle éventuellement substitué, t-butyle ou un groupe phényle éventuellement substitué.

**8.** Procédé selon la revendication 7, où R est le groupe t-butyle.

**9.** Procédé selon l'une quelconque des revendications 1 à 8, où X est un atome de chlore ou un atome de brome.

**10.** Procédé selon l'une quelconque des revendications 1 à 9, où l'halogénure de métal d'acide de Lewis est le bromure de zinc.

**11.** Procédé selon l'une quelconque des revendications 1 à 10, où l'halogénure d'acide représenté par la formule (V) est le bromure de pivaloyle.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2005012326 A
- WO 2011047113 A
- WO 2012140120 A


### Non-patent literature cited in the description


- **KNOCHEL, P. et al.** Stereoselective C-Glycosylation Reactions with Arylzinc Reagents. *Organic Letters,* 2012, vol. 14 (6), 1480-1483
- **TIWARI, P. et al.** Acylation of carbohydrates over Al2O3: preparation of partially and fully acylated carbohydrate derivatives and acetylated glycosyl chlorides. *Carbohydrate Research,* 2006, vol. 341, 339-350
- **GRYNKIEWICZ, G. et al.** Direct transformation of methyl glycopyranosides into corresponding Glycosyl Halides. *Polish J. Chem.,* 1987, vol. 61, 149-153
- **POLAT, T. et al.** Zinc triflate-benzoyl bromide: a versatile reagent for the conversion of ether into benzoate protecting groups and ether glycosides into glycosyl bromides. *Carbohydrate Research,* 2003, vol. 338, 447-449
- **SANTOSH, K. G.** Indium(III) triflate-mediated one-step preparation of Glycosyl halides from free sugars. *Synthetic Communications,* 2010, vol. 40, 3378-3383
- *Organic Letters,* 2012, vol. 14 (6), 1480-1483